(19) Europäisches Patentamt
European Patent Office
Office européen des brevets

(11) Veröffentlichungsnummer : **0 033 529**
**B1**

(12) # EUROPÄISCHE PATENTSCHRIFT

(45) Veröffentlichungstag der Patentschrift : **26.09.84**

(51) Int. Cl.[3] : **C 07 D211/58**

(21) Anmeldenummer : **81100698.0**

(22) Anmeldetag : **31.01.81**

(54) **Verfahren zur Herstellung von 4-Amino-2,2,6,6-tetramethylpiperidin.**

(30) Priorität : **02.02.80 DE 3003843**

(43) Veröffentlichungstag der Anmeldung : **12.08.81 Patentblatt 81/32**

(45) Bekanntmachung des Hinweises auf die Patenterteilung : **26.09.84 Patentblatt 84/39**

(84) Benannte Vertragsstaaten : **BE CH DE FR GB IT LI LU NL SE**

(56) Entgegenhaltungen :
**FR-A- 2 074 871**
**FR-A- 2 264 022**
**FR-A- 2 333 795**
**FR-A- 2 370 731**
**FR-A- 2 409 985**
**Journal of Polymer Science 10 (1972), 3306**
**Houben Weyl, Amine (1957), S. 611**

(73) Patentinhaber : **CHEMISCHE WERKE HÜLS AG**
**Postfach 1320**
**D-4370 Marl 1 (DE)**

(72) Erfinder : **Broschinski, Lothar, Dr.**
**Lüenbrink 18**
**D-4760 Werl (DE)**
Erfinder : **Disteldorf, Josef, Dr.**
**Am Sengenhoff 2a**
**D-4690 Herne 1 (DE)**
Erfinder : **Hübel, Werner, Dr.**
**Birnenbruchstrasse 34**
**D-4690 Herne 1 (DE)**
Erfinder : **Kriebel, Günter, Dr.**
**Gaussstrasse 15**
**D-4690 Herne 2 (DE)**

(74) Vertreter : **Steil, Hanna, Dipl.-Chem.**
**RSP PATENTE - PB 15 Postfach 1320**
**D-4370 Marl 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 033 529 B1

Jouve, 18, rue St-Denis, 75001 Paris, France

**Beschreibung**

Die Erfindung betrifft ein neues Verfahren zur Herstellung von 4-Amino-2,2,6,6-tetramethylpiperidin (nachfolgend TAD, « Triacetondiamin », genannt).

Es ist bereits bekannt, TAD entsprechend dem nachfolgenden Formelschema herzustellen :

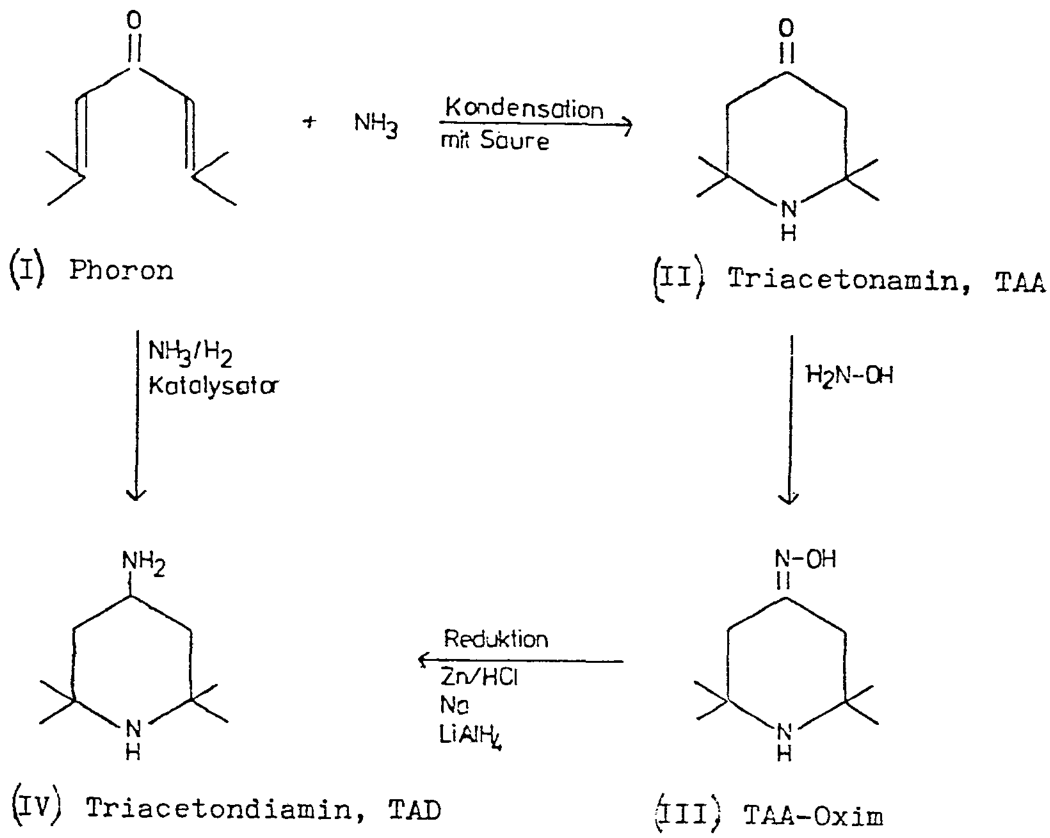


(I) Phoron

(II) Triacetonamin, TAA

(IV) Triacetondiamin, TAD

(III) TAA-Oxim

Dabei wurde stets von Phoron ausgegangen, das zunächst zu Triacetonamin (TAA) kondensiert wurde, dieses mit Hydroxylamin zum Oxim umgesetzt und letzteres schließlich mit geeigneten Reduktionsmitteln zum gewünschten TAD reduziert wurde.

Erst in neuerer Zeit wurde gemäß DE-A-24 12 750 ein einstufiges Verfahren gefunden, in dem Phoron direkt zum TAD in Ausbeuten bis zu 71 % umgesetzt werden kann. Da aber gemäß DE-A-28 07 172 zwischenzeitlich TAA durch Umsetzung von Aceton und Ammoniak im Vergleich zu Phoron technisch und wirtschaftlich günstiger zugänglich ist, besteht ein Interesse zur Synethese von TAD aus TAA auf einfacherem Wege. Dies wurde im Labormaßstab erreicht, s. J. Pol., Sci. 10, 3306 (1972), indem TAA in großem Methanolüberschuß gelöst mit gasförmigem Ammoniak bis zur Sättigung unter Eiskühlung bei 10-15 °C umgesetzt wurde und das Reaktionsgemisch an Raney-Nickel unter milden Bedingungen mit Wasserstoff reduziert wurde, wobei TAD nach destillativer Aufarbeitung in 99 %iger Ausbeute anfiel ; Angaben über die Reinheit fehlen. Eine solche Herstellungsweise kann großtechnisch noch nicht befriedigen, weshalb es Aufgabe dieser Erfindung war, einen solchen Syntheseweg zur Erzeugung von TAD aus TAA zu suchen.

Es wurde gefunden, daß man TAD in sehr guten bis nahezu quantitativen Ausbeuten erhält, wenn man TAA in Abwesenheit eines Solvens und in Gegenwart von Hydrierkatalysatoren mit einem Überschuß an Ammoniak von 10 bis 50 Mol und Aufdrücken von Wasserstoff bei einem Druck von 50-500 bar hydriert, wobei bei kontinuierlicher Fahrweise die Temperaturen zwischen 120-180 °C bei diskontinuierlicher Fahrweise zwischen 180-220 °C liegen Wasserstoff behandelt. Das erfindungsgemäße Verfahren kann als reduktive Aminierung bezeichnet und durch nachfolgende Reaktionsgleichung schematisch wiedergegeben werden :

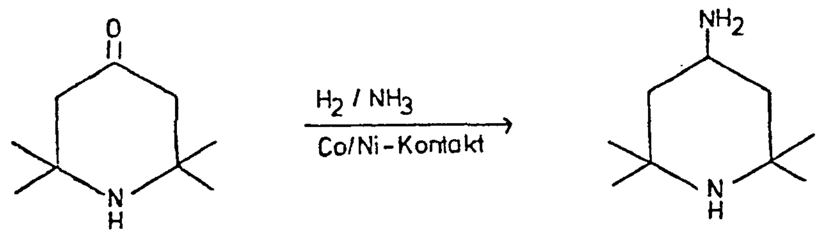

Je Mol TAA wird ein Überschuß von 10-50 Mol $NH_3$ eingesetzt, vorzugsweise 10-30 Mol. Auch weit größere Überschüsse an Ammoniak sind möglich, bringen aber keine weiteren Vorteile und sind deshalb unwirtschaftlich. Der Überschuß an Ammoniak bewirkt eine Unterdrückung von Ausbeute-mindernden Nebenreaktionen, speziell die Bildung von dimeren Produkten mit einer sekundären NH-Gruppe als Brücke zwischen zwei TAA-Resten.

Wasserstoff wird ebenfalls über die stöchiometrisch erforderliche Menge — je ein Mol pro Mol TAA — hinausgehend eingesetzt. Die Wasserstoffmenge wird so bemessen, daß sich der für die Reaktion gewählte Druckbereich von 50-500 bar einstellt. Ein bevorzugter Druckbereich liegt zwischen 200-350 bar.

Das erfindungsgemäße Verfahren wird im Abwesenheit eines Lösungsmittels durchgeführt.

Als Hydrierkatalysatoren können an sich bekannte, handelsübliche Kontakte verwendet werden wie Metalle und Verbindungen derselben aus der 8. Nebengruppe des Periodensystems (Eisen, Kobalt, Nickel, Ruthenium, Rhodium, Palladium, Osmium, Iridium, Platin) sowie Chrom, Mangan, Kupfer, Zink, Molybdän, Wolfram, Rhenium und Kupferchromit.

Insbesonders können die Metalle Kobalt, Nickel, Eisen und die Platinmetalle verwendet werden. Die Metalle können als solche oder auch in Form von Trägerkatalysatoren eingesetzt werden. Als Trägermaterial können die üblichen, Al-oxide, $SiO_2$, Silikate, Kohle und ähnliche dienen.

Die Menge des Katalysators ist nicht wesentlich. Bei kontinuierlicher Durchführung wird im allgemeinen mit einer LHSV von 0,05 bis 0,25, insbesondere 0,1 l/l · h, gerechnet als TAA, gearbeitet, bei diskontinuierlicher Durchführung mit einer Katalysatormenge von 1-10 %, bezogen auf TAA.

Im allgemeinen wird das erfindungsgemäße Verfahren bei diskontinuierlicher Arbeitsweise so durchgeführt, daß man den Katalysator und Ammoniak mit dem TAA zusammen in einem Autoklaven vorlegt und Wasserstoff aufdrückt und auf die gewünschte Reaktionstemperatur aufheizt. Die Reaktionszeit beträgt dann etwa 1-4 Stunden, das Ende der Reaktion ist durch Beendigung der $H_2$-Aufnahme angezeigt und durch GC-Analyse überprüfbar. Bei kontinuierlicher Arbeitsweise werden die Reaktanten durch einen mit Katalysator gefüllten Reaktor geleitet, der auf die gewünschte Reaktionstemperatur und den gewünschten Systemdruck eingestellt ist. Dabei kann nach dem Sumpf- oder Rieselverfahren gearbeitet werden, insbesondere aber im Rieselverfahren.

Der technische Fortschritt des erfindungsgemäßen Verfahrens gegenüber dem Stand der Technik beruht auf der hohen Ausbeute, die bei 95 % liegt, und der einfachen Durchführbarkeit des einstufigen Verfahrens unter Verwendung kostengünstiger Katalysatoren. Außerdem war die Durchführbarkeit des Verfahrens nicht zu erwarten, da TAA ein instabiles Produkt ist, das besonders in Gegenwart von $NH_3$-Überschuß in zahlreiche Nebenprodukte aufspaltet bzw. weiterreagiert ; die bisherigen Synthesen mit TAA als Zwischenprodukt haben diesen Stoffeigenschaften offensichtlich Rechnung getragen.

Beim erfindungsgemäßen Verfahren fällt dagegen überraschend TAD in hoher Ausbeute an, das sich destillativ leicht zu einem Produkt von hoher Reinheit aufarbeiten läßt.

TAD ist ein wertvolles Zwischenprodukt für die Herstellung von Verbindungen, die zur Stabilisierung synthetischer Polymere benötigt werden.

## Beispiel 1 (Vergleich)

In einem Autoklaven werden 155 g TAA, 15 g Co-Katalysator der Firma Hoechst AG mit dem Handelsnamen RCH 45/20 unter $N_2$ eingefüllt, nach dem Schließen des Autoklaven werden 500 ml fl. Ammoniak eingefüllt und Wasserstoff auf 100 bar aufgedrückt. Nach Aufheizen unter Wippen auf 140 °C wurde weiter Wasserstoff auf 300 bar nachgedrückt und von Zeit zu Zeit nach Maßgabe des am Druckabfall erkennlichen Verbrauchs nachgedrückt. Nach Beendigung der $H_2$-Aufnahme wurde noch 1 h auf Druck und Temperatur gehalten, dann abgekühlt und entspannt. Nach Öffnen des Autoklaven konnte ein kristallines Umsetungsprodukt entnommen werden. Laut GC-Untersuchung bestand es fast völlig aus TAA-Aminoalkohol und enthielt nur in geringen Mengen TAD.

## Beispiel 2

Wie in Beispiel 1, jedoch wird die Reaktionstemperatur auf 180-200 °C gesteigert. Nach Beendigung des Versuchs lag ein flüssiges Reaktionsprodukt vor, das nach Abfiltrieren vom Kontakt laut GC zu 95 % aus TAD bestand. Durch Labordestillation konnte eine 99 %ige TAD-Fraktion zu 90 % erhalten werden.

## Beispiel 3

Durch einen Schachtofen, der mit 500 ml Co-Katalysator der Firma Hoechst AG mit dem Handelsnamen RCH 45/20 beschickt war, wurden im Rieselverfahren TAA und $NH_3$ unter $H_2$-Druck durchgesetzt. Als Reaktionstemperatur wurden 130-140 °C durch elektrische Beheizung eingestellt, der Systemdruck mit $H_2$ auf 250 bar unter Aufrechterhaltung eines Gasstromes eingestellt (Abgasfahrweise, 100 l/h Abgas) und 0,1 l/l · h TAA sowie 0,4 l/l · h $NH_3$ durchgesetzt. Nach Abtrennung des überschüssigen Wasserstoffs in einer Abscheiderflasche wurde das Reaktionsgemisch in einer Druckkolonne vom überschüssigem $NH_3$ befreit. Das Sumpfprodukt war leut GC praktisch vollständig umgesetztes TAA und bestand zu $>$ 95 % aus

TAD. Die chargenweise destillative Aufarbeitung im Labor erbrachte Ausbeuten an Rein-TAD in Höhe von 90-95 % bei einer Reinheit von 99,5 % ; die Laborkolonne hatte dabei ca. 40 theor. Böden und es wurde mit einem Rückflußverhältnis von 20 : 1 gearbeitet. Als Nebenprodukte traten laut GC neben einer Reihe nicht identifizierter Peaks in untergeordneten Mengen nur Tetramethylpiperidin, Isopropylamin und Diacetonamin auf.

**Anspruch**

Verfahren zur Herstellung von 4-Amino-2,2,6,6-tetramethyl-piperidin durch Umsetzung von 2,2,6,6-Tetramethyl-piperidin-4-on mit Überschuß Ammoniak und Wasserstoff in Gegenwart von Hydrierkatalysatoren bei erhöhter Temperatur und erhöhtem Druck, dadurch gekennzeichnet, daß in Abwesenheit eines Solvens mit einem Überschuß an Ammoniak von 10 bis 50 Mol und Aufdrücken von Wasserstoff bei einem Druck von 50-500 bar hydriert wird, wobei bei kontinuierlicher Fahrweise die Temperaturen zwischen 120-180 °C, bei diskontinuierlicher Fahrweise zwischen 180-220 °C liegen.

**Claim**

Process for the preparation of 4-amino-2,2,6,6-tetramethyl-piperidine by reacting 2,2,6,6-tetramethyl-piperidin-4-one with excess ammonia and hydrogen in the presence of hydrogenation catalysts at elevated temperature and elevated pressure, characterised in that hydrogenation is effected in the absence of a solvent, with an excess of ammonia of 10 to 50 moles and with hydrogen being forced in at a pressure of 50-500 bar, the temperatures being between 120-180 °C if the process is carried out continuously and between 180-220 °C if it is carried out discontinuously.

**Revendication**

Procédé pour la fabrication de 4-amino-2,2,6,6-tétraméthylpipéridine par réaction de 2,2,6,6-tétraméthylpipéridine-4-one avec un excès de gaz ammoniac et d'hydrogène, en présence de catalyseurs d'hydrogénation et sous une pression élevée, procédé caractérisé en ce que l'on assure l'hydrogénation en l'absence de solvant avec un excès de 10 à 50 moles de gaz ammoniac, et introduction d'hydrogène sous une pression de 50 à 500 bars, les températures se situant, si l'on opère en continu entre 120 et 180 °C, et si l'on opère d'une façon discontinue, entre 180 et 220 °C.